(19) **Europäisches Patentamt / European Patent Office / Office européen des brevets**

(11) **EP 3 928 094 B1**

(12) **FASCICULE DE BREVET EUROPEEN**

(45) Date de publication et mention de la délivrance du brevet:
**03.12.2025 Bulletin 2025/49**

(21) Numéro de dépôt: **20712389.4**

(22) Date de dépôt: **25.03.2020**

(51) Classification Internationale des Brevets (IPC):
**G01N 33/00** *(2006.01)*

(52) Classification Coopérative des Brevets (CPC):
**G01N 33/0047**

(86) Numéro de dépôt international:
**PCT/EP2020/058402**

(87) Numéro de publication internationale:
**WO 2020/193647 (01.10.2020 Gazette 2020/40)**

(54) **PROCÉDÉ POUR DÉTERMINER UN EMPOISONNEMENT POTENTIEL D'UN CAPTEUR D'UN NEZ ÉLECTRONIQUE PAR UN COMPOSÉ VOLATIL**

VERFAHREN ZUR BESTIMMUNG EINER MÖGLICHEN VERGIFTUNG EINES SENSORS EINER ELEKTRONISCHEN NASE DURCH EINE FLÜCHTIGE VERBINDUNG

METHOD FOR DETERMINING A POTENTIAL POISONING OF A SENSOR OF AN ELECTRONIC NOSE BY A VOLATILE COMPOUND

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.03.2019 FR 1903287**

(43) Date de publication de la demande:
**29.12.2021 Bulletin 2021/52**

(73) Titulaire: **Aryballe**
**38000 Grenoble (FR)**

(72) Inventeurs:
- **LIVACHE, Thierry**
  **38540 JARRIE (FR)**
- **HERRIER, Cyril**
  **38000 GRENOBLE (FR)**
- **DUBREUIL, Romain**
  **38000 GRENOBLE (FR)**

(74) Mandataire: **Santarelli**
**Tour Trinity**
**1 bis Place de la Défense**
**92400 Courbevoie (FR)**

(56) Documents cités:
**WO-A1-2004/010085     FR-A1- 3 071 061**
**US-A1- 2002 146 352**

- **JOHN-ERIK HAUGEN ET AL: "A calibration method for handling the temporal drift of solid state gas-sensors", ANALYTICA CHIMICA ACTA, vol. 407, no. 1-2, 29 February 2000 (2000-02-29), pages 23 - 39, XP055152962, ISSN: 0003-2670, DOI: 10.1016/S0003-2670(99)00784-9**
- **DISTANTE C ET AL: "Support vector machines for olfactory signals recognition", SENSORS AND ACTUATORS B: CHEMICAL, ELSEVIER BV, NL, vol. 88, no. 1, 1 January 2003 (2003-01-01), pages 30 - 39, XP004395009, ISSN: 0925-4005, DOI: 10.1016/S0925-4005(02)00306-4**

**EP 3 928 094 B1**

# Description

## Domaine technique

L'invention relève du domaine des nez électroniques.

Plus spécifiquement, l'invention se rapporte à un procédé pour déterminer un empoisonnement potentiel d'un capteur d'un nez électronique par un composé volatil suite à une exposition du capteur à un échantillon gazeux comprenant au moins ce composé volatil et, si empoisonnement il y a, pour déterminer si le capteur est encore fonctionnel, c'est-à-dire s'il est encore capable de réaliser une mesure fiable, ou, au contraire, s'il est saturé et ne doit plus être utilisé.

L'invention se rapporte également à un programme d'ordinateur comprenant des instructions pour la mise en œuvre par un ordinateur de certaines étapes de ce procédé ainsi qu'à un nez électronique comprenant une unité de traitement de données pour la mise en œuvre desdites étapes.

Le procédé de l'invention peut être utilisé dans tout type de nez électronique. Il présente, toutefois, un intérêt tout particulier pour les nez électroniques dits bio-inspirés, qui comprennent un réseau de capteurs dont la partie sensible (c'est-à-dire la partie des capteurs qui interagit physico-chimiquement avec les composés volatils) est fonctionnalisée par des récepteurs biologiques ou biomimétiques, tels que celui décrit par S. Brenet et al. dans Analytical Chemistry 2018, 90, 9879-9887.

## État de la technique antérieure

L'olfaction, ou odorat, est le sens dont sont dotés les mammifères et, notamment, l'homme et qui leur permet de détecter et d'analyser les composés volatils présents dans un milieu gazeux et, notamment, l'air ambiant.

Aujourd'hui, il existe une très forte demande en instruments portables qui soient capables de mimer l'olfaction. Ces instruments, qui sont appelés nez électroniques, ont potentiellement de très nombreuses applications et notamment :

- dans le domaine de la santé, par exemple pour offrir un substitut à l'odorat à des personnes souffrant d'une perte de l'odorat, partielle (hyposmie) ou totale (anosmie), ou pour diagnostiquer ou suivre l'évolution de maladies qui s'accompagnent de la présence de composés volatils odorants dans les fluides biologiques ou dans l'haleine, telles que le diabète, certains cancers (cancers de la prostate, du poumon, cancer du poumon, cancer ovarien, ...) et certaines infections microbiennes ;
- dans les industries alimentaire, cosmétique et pharmaceutique, par exemple pour détecter d'éventuelles contaminations dans les chaînes de fabrication et/ou de distribution ou pour effectuer des contrôles qualité sur les matières premières ou les produits finis ;
- dans le domaine des arômes et des parfums ;
- dans le domaine de la sécurité des biens et des personnes, par exemple pour surveiller des sites fabriquant, stockant, manipulant et/ou susceptibles d'être contaminés par des matières volatiles potentiellement dangereuses, pour détecter la présence de substances dangereuses telles que des explosifs ou des agents toxiques, ou de substances illicites telles que des stupéfiants, ou encore pour rechercher des personnes ensevelies sous des décombres ou des éboulis ; et
- dans le domaine de l'environnement, par exemple pour le suivi de la qualité de l'air ou d'ambiances plus ou moins confinées, ou pour la surveillance et l'analyse de nuisances olfactives et, notamment, d'odeurs dues à des composés organiques volatils soufrés ou aminés, d'origine industrielle ou agricole.

Comme son analogue biologique, un nez électronique se compose principalement de trois systèmes, à savoir :

(1) un système fluidique pour le transport d'un échantillon gazeux de l'extérieur du nez électronique vers l'intérieur de ce nez, ainsi que le rinçage du système par un flux gazeux neutre.

(2) un système de détection qui comprend un réseau de capteurs à réactivité croisée vis-à-vis des composés volatils présents dans un échantillon du milieu gazeux, les capteurs jouant le rôle des récepteurs olfactifs du nez humain ; et

(3) un système informatique assurant le traitement des réponses émises par les capteurs sous forme de signaux, ce système jouant le rôle du cerveau humain.

Les capteurs d'un nez électronique doivent satisfaire à un certain nombre d'exigences dont celle de présenter, vis-à-vis des composés volatils, une grande sensibilité et une sélectivité mais aussi celle de présenter une stabilité, à savoir que, dans des conditions fixes, la ligne de base des capteurs doit rester constante ou quasi constante, et une réversibilité, à savoir que les capteurs doivent être capables de revenir à leur état initial en l'absence de toute exposition à un composé volatil.

Or, il s'avère que certains composés volatils tels que les composés soufrés (en particulier, le sulfure d'hydrogène et les thiols tels que l'éthanethiol) et les composés aminés - qui sont des cibles d'intérêt dans de nombreux domaines d'application des nez électroniques - ainsi que certains composés issus de la fumée de cigarette s'adsorbent de manière prolongée sur la partie sensible des capteurs qui réagissent à ces composés. Il s'ensuit une modification de l'état de surface de la partie sensible des capteurs avec, à la clé, une baisse de leur sensibilité et, donc, de la reproductibilité des mesures effectuées par les capteurs lorsqu'ils sont exposés plusieurs fois de suite aux composés volatils en question, cette baisse étant d'autant plus prononcée que la quantité de composés volatils adsorbée est élevée.

On parle d'empoisonnement des capteurs et les composés volatils responsables de cet empoisonnement sont communément appelés composés volatils poisons.

L'empoisonnement ne serait-ce que de quelques capteurs du réseau de capteurs d'un nez électronique affecte les performances globales de ce nez.

Certaines techniques ont été décrites pour diminuer cet effet d'empoisonnement. Citons, par exemple la demande de brevet français 3 071 061, qui propose d'utiliser un revêtement perfluoré pour diminuer de façon générale la dérive des capteurs qui n'est pas uniquement liée aux composés poisons (dégradation des capteurs, variation d'humidité ou environnementale, etc.).

Il se trouve qu'à ce jour, il n'existe pas d'outil qui permette de déterminer si un ou plusieurs capteurs du réseau de capteurs d'un nez électronique sont ou non empoisonnés et, si c'est le cas, de savoir si ce ou ces capteurs sont encore capables de réaliser des mesures fiables ou si leur degré d'empoisonnement est tel qu'il nécessite d'être inactivés.

Or, il serait souhaitable de pouvoir disposer d'un tel outil pour optimiser les performances des nez électroniques et, notamment, des nez électroniques bioinspirés.

US-A-2002/0146352 décrit un procédé qui permet d'alerter l'utilisateur d'un capteur de gaz combustibles à billes catalytiques que ce capteur est empoisonné et dans lequel un signal d'alerte est émis lorsque la dérive de la ligne de base du capteur atteint un niveau prédéterminé dans un laps de temps donné.

**Exposé de l'invention**

L'invention vise justement à remédier aux manques de l'état de la technique en proposant, tout d'abord, un procédé qui permet de déterminer de manière simple et sûre un empoisonnement potentiel d'un capteur d'un nez électronique par un composé volatil suite à une $n^{ième}$ exposition du capteur à un échantillon gazeux comprenant au moins ce composé volatil, n étant un entier supérieur ou égal à 1.

Ce procédé comprend les étapes consistant à :

a) éliminer l'échantillon gazeux du capteur après l'exposition du capteur à l'échantillon gazeux ;
b) mesurer la ligne de base $B_n$ du capteur après l'élimination de l'échantillon gazeux ;
c) calculer un indicateur d'empoisonnement IE par la formule :

$$IE = (B_n - B_{n-1}) - (f \times U_n)$$

dans laquelle :

$B_{n-1}$ est la ligne de base du capteur avant l'exposition du capteur à l'échantillon gazeux ;
$U_n$ est l'amplitude du signal émis par le capteur lors de son exposition à l'échantillon gazeux ;
f est un paramètre de pondération ; et

d) comparer l'indicateur d'empoisonnement IE ainsi calculé à une valeur seuil S1;
moyennant quoi :

- si IE est inférieur à la valeur seuil S1, alors le capteur n'est pas empoisonné ; et
- si IE est supérieur ou égal à la valeur seuil S1, alors le capteur est empoisonné ; et comprend de plus, si IE est égal ou supérieur à la valeur seuil S1, les étapes consistant à :

e) calculer un indicateur de fonctionnalité IF du capteur par la formule :

$$IF = \frac{B_n - B}{p}$$

dans laquelle :

B est une ligne de base de référence ;
p est un paramètre représentatif de la gamme de travail du capteur ; et

f) comparer l'indicateur de fonctionnalité IF ainsi calculé à une valeur seuil S2 ; moyennant quoi :

- si IF est inférieur à la valeur seuil S2, alors le capteur est encore fonctionnel, et
- si IF est supérieur ou égal à la valeur seuil S2, alors le capteur n'est plus fonctionnel.

Dans ce qui précède et ce qui suit, on entend par « capteur », un ensemble qui comprend :

* une partie sensible qui est disposée sur un substrat et qui est fonctionnalisée par un ou plusieurs récepteurs capables d'interagir de façon physico-chimique avec au moins un composé volatil, et
* un système de mesure, typiquement appelé transducteur, dont la fonction est de mesurer une variation d'une grandeur physique résultant de l'interaction physico-chimique récepteur(s)/composé volatil et de convertir cette mesure en un signal mesurable, étant entendu que, dans le cas d'un réseau de capteurs comme celui que comprend un nez électronique, chacun des capteurs de ce réseau peut comprendre son propre système de mesure ou partager avec d'autres capteurs un système de mesure qui leur est commun.

Par ailleurs, on entend par « ligne de base » d'un capteur, plus communément appelée par son équivalent anglais « baseline », l'intensité du signal émis par le capteur en l'absence de tout contact de la partie sensible de ce capteur avec un composé volatil auquel le capteur est susceptible de réagir.

Comme connu en soi, l'étape d'élimination de l'échantillon gazeux du capteur, ou étape a), est avantageusement réalisée par rinçage du capteur avec un gaz neutre, lequel peut notamment être l'air ambiant, de l'air sec, de l'air humide à l'humidité contrôlée, de l'hélium, du diazote, de l'argon ou encore du dioxyde de carbone.

Conformément à l'invention, la valeur seuil S1 est, de préférence, égale à 0, auquel cas le paramètre de pondération f est positif et inférieur à 1, préférentiellement au plus égal à 0,5. Mieux encore, f est égal à 0,05.

Par ailleurs, un capteur qui est empoisonné par un composé volatil peut :

> \* soit être encore fonctionnel, à savoir qu'il peut encore servir à réaliser une ou plusieurs mesures parce que, bien qu'empoisonné, il n'est pas saturé par le composé volatil,
> \* soit ne plus être fonctionnel du fait d'une saturation par le composé volatil.

Les étapes e) et f) précitées du procédé de l'invention permettent justement de vérifier, dans le cas où le capteur est empoisonné, s'il est encore fonctionnel ou non.

L'expression « gamme de travail » du capteur s'entend dans son acceptation habituelle, à savoir qu'elle désigne l'intervalle de valeurs de la grandeur physique devant être mesurée par le capteur (telle qu'une variation de réflectivité dans le cas d'un capteur optique à résonance des plasmons de surface) dans lequel le capteur est apte à fournir des mesures fiables. Tout capteur présente une gamme de travail qui est indiquée par son fabricant.

Conformément à l'invention, la ligne de base B est, de préférence, la ligne de base initiale $B_0$ du capteur, c'est-à-dire la ligne de base que présente ce capteur :

- soit à l'état neuf,
- soit lorsque, après avoir allumé le nez électronique, réalisé les tests de calibration préconisés par son constructeur et rincé le capteur au moyen d'un gaz neutre pendant un temps allant de 30 secondes à 2 minutes et, typiquement, pendant 1 minute, aucune variation de signal n'est observée pour le capteur.

Par ailleurs, p est, de préférence, la limite supérieure, notée Γ, de la gamme de travail du capteur.

Quant à la valeur seuil S2, elle va, de préférence, de 0,4 à 0,9 et, mieux encore, est égale à 0,7.

Conformément à l'invention, le ou les récepteurs avec lequel ou lesquels la partie sensible du capteur est fonctionnalisée sont, de préférence, des biomolécules, c'est-à-dire des molécules présentes naturellement chez les êtres vivants telles que des oligonucléotides, des acides nucléiques, des glucides, des peptides, des protéines, des lipides, etc, ou des molécules biomimétiques, c'est-à-dire des molécules qui imitent structurellement et/ou fonctionnellement les molécules biologiques.

Par ailleurs, le composé volatil peut être un composé organique, ou COV, ou un composé inorganique tel que l'ammoniac, le sulfure d'hydrogène, le dioxyde de soufre, etc.

À cet égard, on précise que la notion de COV est définie par la Directive 1999/13/CE du Conseil Européen du 11 mars 1999 en vertu de laquelle :

- un composé organique volatil est *« tout composé organique ayant une pression de vapeur de 0,01 kPa (soit 9,87.10⁻⁵ atm) ou plus à une température de 293,15 K (soit 20°C) ou ayant une volatilité correspondante dans les conditions d'utilisation particulières »* (cf. paragraphe 17 de l'article 2 de la Directive) ;
- un composé organique est *« tout composé comprenant au moins l'élément carbone et un ou plusieurs des éléments suivants : hydrogène, halogènes, oxygène, soufre, phosphore, silicium ou azote, à l'exception des oxydes de carbone et des carbonates et bicarbonates inorganiques »* (cf. paragraphe 16 de l'article 2 de la Directive).

Ainsi, sont notamment des COV, certains hydrocarbures acycliques saturés ou insaturés (éthane, propane, n-butane, n-hexane, éthylène, propylène, 1,3-butadiène, acétylène, etc), certains hydrocarbures cycliques non aromatiques saturés ou insaturés (cyclopropane, cyclopentane, cyclohexane, etc), certains hydrocarbures aromatiques (benzène, toluène, xylènes, éthylbenzène, etc), certains hydrocarbures halogénés (dichlorométhane, trichlorométhane, chloroéthane, trichloroéthylène, tétrachloro-éthylène, etc), certains alcools (méthanol, éthanol, 1-propanol, 2-propanol, éthylène glycol, propylène glycol, etc), certains aldéhydes (formaldéhyde, acétaldéhyde, propanal, 2-propenal (ou acroléine), etc), certaines cétones (acétone, méthyléthylcétone, 2-butanone, méthylvinylcétone, etc), certains esters (acétate de méthyle, acétate d'éthyle, acétate d'isopropyle, butyrate d'isoamyle, etc), certains éthers (éther diéthylique, n-butyléther d'éthylène glycol, 1,4-dioxane, etc), certains acides (acide acétique, acide propanoïque, etc), certaines amines (éthylamine, diméthylamine, triméthylamine, diéthylamine, amylamine, etc), certains amides (diméthylformamide par exemple), certains thiols (mercaptan méthylique ou méthanethiol, mercaptan éthylique ou éthanethiol, etc), certains nitriles (acétonitrile, acrylonitrile, etc) ainsi que d'autres composés comprenant plusieurs fonctions chimiques différentes.

L'invention a également pour objet un programme d'ordinateur comprenant des instructions qui, lorsque le programme est exécuté par un ordinateur, conduisent l'ordinateur à mettre en œuvre les étapes de calcul et de comparaison du procédé tel que précédemment défini.

L'invention a encore pour objet un nez électronique comprenant une unité de traitement de données configurée pour mettre en œuvre les étapes de calcul et de comparaison du procédé tel que précédemment défini.

D'autres caractéristiques et avantages de l'invention ressortiront du complément de description qui suit et qui est donné en référence aux figures annexées.

Il va de soi toutefois que ce complément de description n'est donné qu'à titre d'illustration de l'objet de l'invention et ne doit en aucun cas être interprété comme une limitation de cet objet.

**Brève description des figures**

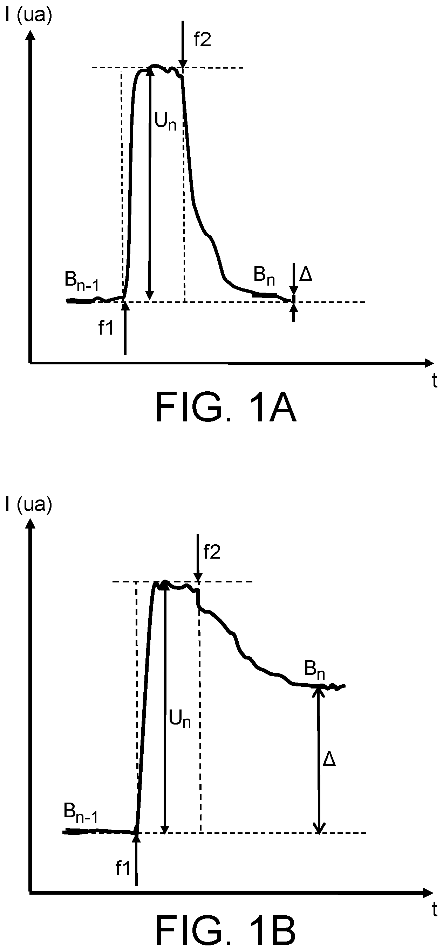

La figure 1A illustre un exemple du profil que présente le signal émis par un capteur de nez électronique avant, pendant et après une exposition à un échantillon gazeux comprenant un composé volatil auquel le capteur est capable de réagir, en l'absence d'empoisonnement du capteur par le composé volatil ; sur cette figure, l'intensité du signal, notée I et exprimée en unités arbitraires (ua) est indiquée sur l'axe des ordonnées, tandis que le temps, noté t, est indiqué sur l'axe des abscisses.

La figure 1B illustre un exemple du profil que présente le signal émis par un capteur de nez électronique avant, pendant et après une exposition à un échantillon gazeux comprenant un composé volatil auquel le capteur est capable de réagir, en cas d'empoisonnement du capteur par le composé volatil ; sur cette figure, l'intensité du signal, notée I et exprimée en unités arbitraires (ua) est indiquée sur l'axe des ordonnées, tandis que le temps, noté t, est indiqué sur l'axe des abscisses.

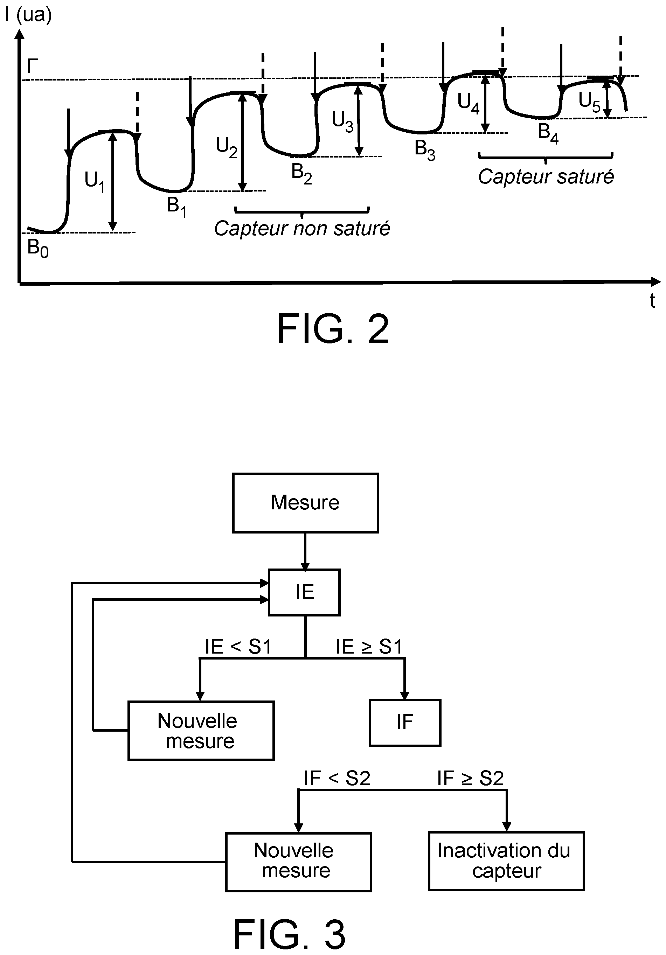

La figure 2 illustre un exemple du profil que présente le signal émis par un capteur de nez électronique lorsqu'il est soumis à une série d'expositions à des échantillons gazeux comprenant un composé volatil auquel le capteur est capable de réagir, en cas d'empoisonnement répété du capteur par le composé volatil allant jusqu'à la saturation de ce capteur ; sur cette figure, l'intensité du signal, notée I et exprimée en unités arbitraires (ua) est indiquée sur l'axe des ordonnées, tandis que le temps, noté t, est indiqué sur l'axe des abscisses.

La figure 3 illustre, sous la forme d'un arbre décisionnel, un exemple de mise en œuvre du procédé de l'invention dans un nez électronique.

**Exposé détaillé de modes de mise en œuvre particuliers**

**I - Empoisonnement et saturation d'un capteur par un composé volatil poison :**

On se réfère tout d'abord aux figures 1A et 1B qui chacune illustrent un exemple du profil que présente le signal émis par un capteur de nez électronique avant, pendant et après une $n^{ième}$ exposition à un échantillon gazeux comprenant un composé volatil auquel le capteur est capable de réagir, n étant en entier supérieur ou égal à 1, en l'absence d'empoisonnement du capteur par le composé volatil pour la figure 1A et dans le cas d'un empoisonnement du capteur par le composé volatil pour la figure 1B.

Sur ces figures, les flèches f1 indiquent le début de l'exposition du capteur à l'échantillon gazeux, par exemple par injection de cet échantillon dans la chambre du nez électronique, tandis que les flèches f2 indiquent le début de l'élimination de l'échantillon gazeux de la chambre du nez électronique, par exemple par injection d'un gaz neutre (air ambiant, air sec, azote, argon, etc) dans cette chambre.

Comme le montrent les figures 1A et 1B, le capteur présente, avant l'exposition à l'échantillon gazeux, une ligne de base, notée $B_{n-1}$, qui correspond à l'intensité du signal que le capteur émet en l'absence de tout contact de sa partie sensible avec un composé volatil auquel il est susceptible de réagir.

L'exposition du capteur à l'échantillon gazeux provoque une augmentation immédiate de l'intensité du signal émis par le capteur. Cette intensité atteint un plateau auquel elle se maintient jusqu'au début de l'élimination de l'échantillon gazeux. L'amplitude du signal, notée $U_n$, est donnée par la différence entre l'intensité du signal au plateau et la ligne de base $B_{n-1}$.

Du fait de l'élimination de l'échantillon gazeux, l'intensité du signal émis par le capteur chute jusqu'à atteindre une nouvelle ligne de base, notée $B_n$.

En l'absence d'empoisonnement du capteur par le composé volatil, la ligne de base $B_n$ est identique à la ligne de base $B_{n-1}$ ou, comme illustré sur la figure 1A, ne diffère de la ligne de base $B_{n-1}$ que d'un faible delta ($\Delta$).

Par contre, comme illustré sur la figure 1B, un empoisonnement du capteur par le composé volatil se traduit par un delta ($\Delta$) entre les lignes de base $B_n$ et $B_{n-1}$ qui est bien supérieur à celui observé en l'absence d'empoisonnement du capteur, ce $\Delta$ pouvant aller jusqu'à 100% de l'amplitude $U_n$ du signal émis par le capteur au cours de son exposition à l'échantillon gazeux.

On se réfère à présent à la figure 2 qui illustre un exemple du profil que présente le signal émis par un capteur de nez électronique lorsqu'il est soumis à une série d'expositions à des échantillons gazeux comprenant un composé volatil auquel le capteur est capable de réagir, en cas d'empoisonnement répété du capteur par le composé volatil allant jusqu'à la saturation de ce capteur.

De manière arbitraire et purement illustrative, les expositions du capteur sont au nombre de cinq sur la figure 2.

Sur cette figure, les flèches directionnelles en trait plein indiquent le début des expositions du capteur aux échantillons gazeux tandis que les flèches directionnelles en pointillés indiquent le début de l'élimination des échantillons gazeux de la chambre du nez électronique.

La ligne de base que présente initialement le capteur, c'est-à-dire avant la première exposition du capteur, est notée $B_0$, tandis que la ligne de base que présente le capteur après chacune des cinq expositions est notée respectivement $B_1$, $B_2$, $B_3$, $B_4$ et $B_5$.

De manière analogue, l'amplitude du signal émis par le capteur au cours de chacune des cinq expositions est respectivement notée $U_1$, $U_2$, $U_3$, $U_4$ et $U_5$.

La ligne en pointillés notée $\Gamma$ indique, quant à elle, la limite supérieure de la gamme de travail du capteur, cette gamme de travail correspondant à l'intervalle de valeurs de la grandeur physique devant être mesurée par le capteur dans lequel le capteur est apte à fournir des mesures fiables.

En attribuant la valeur 0 à la ligne de base $B_0$, le capteur est considéré comme fonctionnel tant que la grandeur physique mesurée varie entre 0 et $\Gamma$ ou, en d'autres termes, le signal émis par le capteur s'inscrit dans l'intervalle [0 ; $\Gamma$].

Comme le montre la figure 2, des expositions successives d'un capteur à des échantillons gazeux comprenant un composé volatil poison se traduisent par une augmentation progressive, exposition après exposition, de la ligne de base du capteur avec, en corollaire, une diminution de l'amplitude du signal émis par le capteur au cours des expositions.

Ainsi, par exemple, $U_2$ est égal à 90 % de $U_1$; $U_3$ est égal à 60 % de $U_1$; $U_4$ est égal à 40 % de $U_1$ tandis que $U_5$ ne représente plus que 20 % de $U_1$.

L'amplitude du signal émis par le capteur au cours des trois premières expositions s'inscrit dans l'intervalle [0 ; $\Gamma$], ce qui signifie que, bien que le capteur soit empoisonné (compte-tenu du $\Delta$ existant entre $B_1$ et $B_0$), il est encore fonctionnel.

Par contre, le signal émis par le capteur au cours de la 4ème exposition sort de l'intervalle [0 ; $\Gamma$], ce qui signifie que le capteur n'est plus fonctionnel car saturé par le composé volatil poison et que les mesures fournies par ce capteur ne sont plus fiables.

C'est justement pour éviter qu'un capteur et, partant, le nez électronique qui comprend ce capteur ne fournissent des mesures non fiables qu'il est proposé selon l'invention :

- d'une part, de déterminer si un capteur est ou non empoisonné par un composé volatil suite à une exposition à un échantillon gazeux comprenant ce composé volatil en calculant un indicateur d'empoisonnement IE et en comparant cet indicateur à une valeur seuil S1 ; et
- d'autre part, dans le cas où il résulte du calcul précédent que le capteur est empoisonné, de déterminer si le capteur est encore fonctionnel ou non en calculant un indicateur de fonctionnalité IF et en comparant cet indicateur à une valeur seuil S2.

## II - Indicateur d'empoisonnement IE d'un capteur :

Si l'on se réfère à nouveau aux figures 1A et 1B, l'indicateur d'empoisonnement IE correspond au $\Delta$ entre les lignes de base $B_n$ et $B_{n-1}$ duquel on soustrait l'amplitude $U_n$ du signal émis par le capteur pendant qu'il est exposé à l'échantillon gazeux, cette amplitude étant pondérée par un paramètre de pondération f.

En d'autres termes, IE est égal à :

$$(B_n - B_{n-1}) - (f \times U_n).$$

Ainsi, si IE est inférieur à la valeur seuil S1, alors le capteur n'est pas empoisonné tandis que, si IE est supérieur ou égal à la valeur seuil S1, alors le capteur est empoisonné.

La valeur seuil S1 est préférentiellement égale à 0.

Le paramètre de pondération f est alors positif et inférieur à 1, avantageusement au plus égal à 0,5. Mieux encore, f est égal à 0,05.

Le profil montré sur la figure 1A est typiquement un profil pour lequel, pour un paramètre de pondération f de 0,05, IE est inférieur à 0, signant ainsi une absence d'empoisonnement du capteur par le composé volatil, tandis que le profil montré sur la figure 1B est typiquement un profil pour lequel, avec le même paramètre de pondération, IE est supérieur à 0, signant, au contraire, un empoisonnement du capteur par le composé volatil.

Si l'on se réfère à la figure 2, l'indicateur d'empoisonnement IE du capteur peut être mesuré dès la première exposition de ce capteur à un échantillon gazeux, auquel cas IE est égal à : $(B_1 - B_0) - (f \times U_1)$ et, si l'on prend f = 0,05, alors IE est égal à $(B_1 - B_0) - 0{,}05U_1$.

## III - Indicateur de fonctionnalité IF d'un capteur empoisonné :

L'indicateur de fonctionnalité IF correspond au $\Delta$ entre la ligne de base $B_n$ - dont on rappelle qu'il s'agit de la ligne de base à laquelle revient le capteur suite à une exposition à un échantillon gazeux - et une ligne de base de référence, notée B, divisé par un paramètre, noté p, représentatif de la gamme de travail du capteur.

En d'autres termes, IF est égal à : $(B_n - B)/p$.

Ainsi, si IF est inférieur à la valeur seuil S2, alors le capteur, bien qu'empoisonné, est encore fonctionnel et peut encore être utilisé pour au moins une mesure tandis que si IF est supérieur ou égal à la valeur seuil S2, alors le capteur est saturé par le composé volatil et ne peut plus être utilisé, au moins de manière temporaire.

La ligne de base B est, de préférence, la ligne de base initiale, notée $B_0$ du capteur.

Le paramètre p est, de préférence, la limite supérieure de la gamme de travail du capteur.

La valeur seuil S2 est, quant à elle, comprise entre 0,4 et 0,9 et, préférentiellement égale à 0,7.

Si l'on se réfère à nouveau à la figure 2, on constate que, pour p = Γ et S2 = 0,7, l'indicateur de fonctionnalité obtenu après la première exposition du capteur à un échantillon gazeux, à savoir IF = (B$_1$ - B$_0$)/Γ, est inférieur à 0,7 ou, autrement dit, à 70 % de l'intervalle [0 ; Γ].

Il en est de même pour l'indicateur de fonctionnalité obtenu après la deuxième exposition du capteur à un échantillon gazeux, à savoir IF = (B$_2$ - B$_0$)/Γ. Le capteur est donc encore fonctionnel après cette deuxième exposition.

Par contre, l'indicateur de fonctionnalité obtenu après la troisième exposition du capteur à un échantillon gazeux, à savoir IF = (B$_3$ - B$_0$)/Γ, est supérieur à 0,7, ce qui signifie que le capteur n'est plus en mesure d'effectuer une nouvelle mesure fiable.

Il en est *a fortiori* de même pour l'indicateur de fonctionnalité obtenu après les quatrième et cinquième expositions du capteur à un échantillon gazeux.

## IV - Schéma d'un mode de mise en œuvre du procédé de l'invention dans un nez électronique :

On se réfère à la figure 3 qui illustre, sous la forme d'un arbre décisionnel, un exemple de mise en œuvre du procédé de l'invention dans un nez électronique.

Le point de départ de cet arbre décisionnel est une mesure ayant été réalisée par un capteur au cours d'une exposition de ce capteur à un échantillon gazeux comprenant un composé volatil auquel ce capteur est susceptible de réagir.

Cette exposition est préférentiellement mais n'est pas nécessairement la première exposition du capteur à un échantillon gazeux comprenant le composé volatil.

Suite à la mesure effectuée par le capteur, l'indicateur d'empoisonnement IE du capteur est calculé et comparé à la valeur seuil S1 par l'unité de traitement de données du nez électronique.

Si IE est inférieur à la valeur seuil S1, alors le nez électronique indique que le capteur est apte à effectuer une nouvelle mesure fiable.

Si IE est supérieur ou égal à la valeur seuil S1, alors l'unité de traitement de données du nez électronique calcule l'indicateur de fonctionnalité IF du capteur et compare cet indicateur à la valeur seuil S2.

Si IF est supérieur ou égal à la valeur seuil S2, alors le nez électronique indique que le capteur n'est plus en mesure d'effectuer de mesures fiables et le capteur est inactivé et destiné à être remplacé.

Si IF est inférieur à la valeur seuil S2, alors le nez électronique indique que le capteur est apte à effectuer une nouvelle mesure fiable.

Si une nouvelle mesure est effectuée, alors l'unité de traitement de données du nez électronique recalcule, au terme de cette mesure, l'indicateur d'empoisonnement IE du capteur et le compare à nouveau à la valeur seuil S1 et, selon le cas, recalcule l'indicateur de fonctionnalité IF et le compare à la valeur seuil S2. Ce schéma est reproduit aussi longtemps que le nez électronique n'indique pas que le capteur n'est plus en mesure d'effectuer une mesure fiable.

## Références citées

S. Brenet et al., Analytical Chemistry 2018, 90, 9879-9887
Demande de brevet français 3 071061
Demande de brevet US 2002/0146352

## Revendications

1. Procédé pour déterminer un empoisonnement potentiel d'un capteur d'un nez électronique par un composé volatil suite à une n$^{\text{ième}}$ exposition du capteur à un échantillon gazeux comprenant au moins ce composé volatil, n étant un entier supérieur ou égal à 1, qui comprend les étapes consistant à :

a) éliminer l'échantillon gazeux du capteur après l'exposition du capteur à l'échantillon gazeux ;
b) mesurer la ligne de base B$_n$ du capteur après l'élimination de l'échantillon gazeux ;
c) calculer un indicateur d'empoisonnement IE par la formule :

$$IE = (B_n - B_{n-1}) - (f \times U_n)$$

dans laquelle :

B$_{n-1}$ est la ligne de base du capteur avant l'exposition du capteur à l'échantillon gazeux ;
U$_n$ est l'amplitude du signal émis par le capteur lors de son exposition à l'échantillon gazeux ;
f est un paramètre de pondération ; et

d) comparer l'indicateur d'empoisonnement IE ainsi calculé à une valeur seuil S1 ;
moyennant quoi :

- si IE est inférieur à la valeur seuil S1, alors le capteur n'est pas empoisonné ; et
- si IE est supérieur ou égal à la valeur seuil S1, alors le capteur est empoisonné ; et qui comprend de plus, si IE est égal ou supérieur à la valeur seuil S1, les étapes consistant à :

e) calculer un indicateur de fonctionnalité IF du capteur par la formule :

$$IF = \frac{B_n - B}{p}$$

dans laquelle :

B est une ligne de base de référence ;
p est un paramètre représentatif de la gamme de travail du capteur ; et

f) comparer l'indicateur de fonctionnalité IF ainsi calculé à une valeur seuil S2 ; moyennant quoi :

- si IF est inférieur à la valeur seuil S2, alors le capteur est encore fonctionnel, et
- si IF est supérieur ou égal à la valeur seuil S2, alors le capteur n'est plus fonctionnel.

**2.** Procédé selon la revendication 1, dans lequel la valeur seuil S1 est égale à 0.

**3.** Procédé selon la revendication 1 ou la revendication 2, dans lequel f est positif et inférieur à 1, de préférence au plus égal à 0,5, et, mieux encore, égal à 0,05.

**4.** Procédé selon l'une quelconque des revendications 1 à 3, dans lequel B est la ligne de base initiale du capteur.

**5.** Procédé selon l'une quelconque des revendications 1 à 4, dans lequel p est la limite supérieure de la gamme de travail du capteur.

**6.** Procédé selon l'une quelconque des revendications 1 à 5, dans lequel la valeur seuil S2 va de 0,4 à 0,9, S2 étant, de préférence, égale à 0,7.

**7.** Procédé selon l'une quelconque des revendications 1 à 6, dans lequel le capteur comprend une partie sensible qui est fonctionnalisée par un ou plusieurs récepteurs capables d'interagir de façon physico-chimique avec le composé volatil, et dans lequel le ou les récepteurs sont choisis parmi des molécules biologiques et des molécules biomimétiques.

**8.** Programme d'ordinateur, comprenant des instructions qui, lorsque le programme est exécuté par un ordinateur, conduisent l'ordinateur à mettre en œuvre les étapes de calcul et de comparaison du procédé selon la revendication 1.

**9.** Nez électronique, comprenant une unité de traitement de données configurée pour mettre en œuvre les étapes de calcul et de comparaison du procédé selon la revendication 1.

**Patentansprüche**

**1.** Verfahren zum Bestimmen einer potenziellen Vergiftung eines Sensors einer elektronischen Nase durch eine flüchtige Verbindung infolge einer n-ten Exposition des Sensors gegenüber einer gasförmigen Probe, die mindestens diese flüchtige Verbindung umfasst, wobei n eine Ganzzahl größer oder gleich 1 ist, das die Schritte umfasst, die aus Folgendem bestehen:

a) Entfernen der gasförmigen Probe aus dem Sensor, nachdem der Sensor der gasförmigen Probe ausgesetzt war;
b) Messen der Sensor-Basislinie $B_n$ nach Entfernung der gasförmigen Probe;
c) Berechnen eines Vergiftungsindikators IE mit folgender Formel:

$$IE = (B_n - B_{n-1}) - (f \times U_n)$$

wobei:

$B_{n-1}$ die Sensor-Basislinie ist, bevor der Sensor der gasförmigen Probe ausgesetzt wird;
$U_n$ die Amplitude des Signals ist, das der Sensor ausgibt, wenn er der gasförmigen Probe ausgesetzt ist;
f ein Gewichtungsparameter ist; und

d) Vergleichen des so berechneten Vergiftungsindikators IE mit einem Schwellenwert S1; wodurch:

- wenn IE kleiner ist als der Schwellwert S1, der Sensor dann nicht vergiftet ist; und
- wenn IE größer oder gleich dem Schwellenwert S1 ist, der Sensor dann vergiftet ist; und das ferner, wenn IE größer oder gleich dem Schwellenwert S1 ist, die Schritte umfasst, die aus Folgendem bestehen:

e) Berechnen eines Funktionsindikators IF des Sensors mit folgender Formel:

$$IF = \frac{B_n - B}{p}$$

wobei:

B eine Referenzbasislinie ist;

p ein Parameter ist, der für den Arbeitsbereich des Sensors repräsentativ ist; und

f) Vergleichen des so berechneten Funktionsindikators IF mit einem Schwellenwert S2; wodurch:

- wenn IF kleiner ist als der Schwellwert S2, der Sensor dann noch funktionsfähig ist, und
- wenn IF größer oder gleich dem Schwellwert S2 ist, der Sensor dann nicht mehr funktionsfähig ist.

**2.** Verfahren nach Anspruch 1, wobei der Schwellenwert S1 gleich 0 ist.

**3.** Verfahren nach Anspruch 1 oder Anspruch 2, wobei f positiv und kleiner als 1 ist, vorzugsweise höchstens gleich 0,5, und besser noch gleich 0,05.

**4.** Verfahren nach einem der Ansprüche 1 bis 3, wobei B die Ausgangsbasislinie des Sensors ist.

**5.** Verfahren nach einem der Ansprüche 1 bis 4, wobei p die obere Grenze des Arbeitsbereichs des Sensors ist.

**6.** Verfahren nach einem der Ansprüche 1 bis 5, wobei der Schwellenwert S2 von 0,4 bis 0,9 reicht, wobei S2 vorzugsweise gleich 0,7 ist.

**7.** Verfahren nach einem der Ansprüche 1 bis 6, wobei der Sensor einen empfindlichen Teil umfasst, der von einem oder mehreren Rezeptoren funktionalisiert ist, die in der Lage sind, physikalisch-chemisch mit der flüchtigen Verbindung zu interagieren, und wobei der oder die Rezeptoren aus biologischen Molekülen und biomimetischen Molekülen ausgewählt sind.

**8.** Computerprogramm, umfassend Anweisungen, die, wenn das Programm von einem Computer ausgeführt wird, den Computer dazu veranlassen, die Berechnungs- und Vergleichsschritte des Verfahrens nach Anspruch 1 durchzuführen.

**9.** Elektronische Nase, umfassend eine Datenverarbeitungseinheit, die so eingerichtet ist, dass sie die Berechnungs- und Vergleichsschritte des Verfahrens nach Anspruch 1 durchführt.

## Claims

**1.** Method for determining a potential poisoning of a sensor of an electronic nose by a volatile compound following a $n^{th}$ exposure of the sensor to a gaseous sample comprising at least this volatile compound, n being an integer greater than or equal to 1, which comprises the steps of:

a) eliminating the gaseous sample from the sensor after the exposure of the sensor to the gaseous sample;
b) measuring the baseline $B_n$ of the sensor after eliminating the gaseous sample;
c) calculating a poisoning indicator IE via formula:

$$IE = (B_n - B_{n-1}) - (f \times U_n)$$

wherein:

$B_{n-1}$ is the baseline of the sensor before the exposure of the sensor to the gaseous sample;
$U_n$ is the amplitude of the signal emitted by the sensor during its exposure to the gaseous sample;
f is a weighting parameter; and

d) comparing the poisoning indicator IE thus calculated with a threshold value S1; whereby:

- if IE is less than the threshold value S1, then the sensor is not poisoned; and
- if IE is greater than or equal to the threshold value S1, then the sensor is poisoned;

and which further comprises, if IE is equal to or greater than the threshold value S1, the steps of:

e) calculating a functionality indicator IF of the sensor via formula:

$$IF = \frac{B_n - B}{p}$$

wherein:

B is a reference baseline;
p is a parameter representative of the working range of the sensor; and

f) comparing the functionality indicator IF thus calculated with a threshold value S2; whereby:

- if IF is less than the threshold value S2, then the sensor is still functional, and
- if IF is greater than or equal to the threshold value S2, then the sensor is no longer functional.

2. Method according to claim 1, wherein the threshold value S1 is equal to 0.

3. Method according to claim 1 or claim 2, wherein f is positive and less than 1, preferably at most equal to 0.5, and, even better, equal to 0.05.

4. Method according to any one of claims 1 to 3, wherein B is the initial baseline of the sensor.

5. Method according to any one of claims 1 to 4, wherein p is the upper limit of the working range of the sensor.

6. Method according to any one of claims 1 to 5, wherein the threshold value S2 ranges from 0.4 to 0.9, S2 being, preferably, equal to 0.7.

7. Method according to any one of claims 1 to 6, wherein the sensor comprises a sensitive portion that is functionalised by one or more receptors capable of interacting physicochemically with the volatile compound, and wherein the receptor or receptors are selected from biological molecules and biomimetic molecules.

8. Computer program, comprising instructions that, when the program is executed by a computer, lead the computer to implement the calculation and comparison steps of the method according to claim 1.

9. Electronic nose, comprising a data processing unit configured to implement the calculation and comparison steps of the method according to claim 1.

FIG. 1A

FIG. 1B

# FIG. 2

# FIG. 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3071061

- US 20020146352 A

**Littérature non-brevet citée dans la description**

- **S. BRENET et al.** *Analytical Chemistry*, 2018, vol. 90, 9879-9887